# EUROPÄISCHE PATENTANMELDUNG

(11) **EP 0 742 197 A1**
(43) Veröffentlichungstag der Anmeldung: **13.11.1996**
(21) Anmeldenummer: 96106896.2
(22) Anmeldetag: 02.05.1996
(51) Int. Cl.: C07C 49/788, C07C 43/275, C07C 255/54, C07C 323/21, C07C 309/66, C09K 19/32

(54) **Derivate des Triphenylens**

(30) Priorität: 11.05.1995 DE 19517208
(71) Anmelder: BASF AKTIENGESELLSCHAFT, 67056 Ludwigshafen (DE)
(72) Erfinder: Paulus, Wolfgang, Dr., 67256 Weisenheim (DE); Etzbach, Karl-Heinz, Dr., 67227 Frankenthal (DE); Rego, James A., Dr., Albuquerque, NM 87108 (US); Schuhmacher, Peter, Dr., 68163 Mannheim (DE); Henderson, Philippe, Dr., 1457 Tourinnes-St.-Lambert (BE); Ringsdorf, Helmut, Prof. Dr., 55124 Mainz (DE); Sandeep, Kumar, Dr., Bangalore 560092, B4-141 R.M.V. Clusters (IN)

(57) **Zusammenfassung**

Derivate des Triphenylens der allgemeinen Formel I 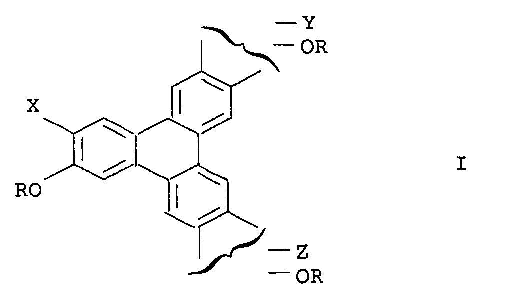 in der die Substituenten folgende Bedeutung haben:
- R: gleiche oder verschiedene C₂-C₂₀-Alkylreste,
- X: Fluor, Chlor, Brom, Jod, Nitro, Cyano, Formyl, eine Azogruppe Ar-N=N-, in der Ar für einen iso- oder heteroaromatischen Rest steht, eine aliphatische Gruppe mit 2 bis 20 C-Atomen oder eine araliphatische Gruppe mit bis zu 20 C-Atomen, die an einem C-Atom oder über Schwefel oder eine Sulfon- oder Sulfonoxygruppierung mit dem Triphenylen verbunden ist
- Y: einen Rest (-OR) oder einen der Reste X
- Z: einen Rest (-OR) oder einen der Reste X,
die Herstellung der Triphenylenderivate sowie deren Verwendung als organische Ladungstransportverbindungen und Photoleiter.

## Beschreibung

Die vorliegende Erfindung betrifft neue Derivate des Triphenylens der allgemeinen Formel I 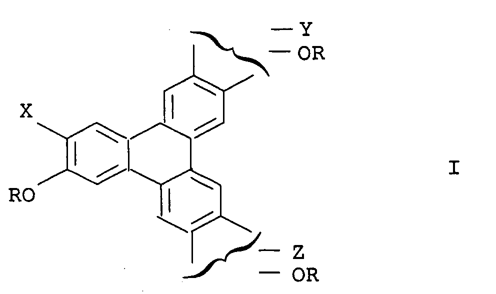 in der die Substituenten folgende Bedeutung haben:
- R: gleiche oder verschiedene C₂-C₂₀-Alkylreste,
- X: Fluor, Chlor, Brom, Jod, Nitro, Cyano, Formyl, eine Azogruppe Ar-N=N-, in der Ar für einen iso- oder heteroaromatischen Rest steht, eine aliphatische Gruppe mit 2 bis 20 C-Atomen oder eine araliphatische Gruppe mit bis zu 20 C-Atomen, die an einem C-Atom oder über Schwefel oder eine Sulfon- oder Sulfonoxygruppierung mit dem Triphenylen verbunden ist
- Y: einen Rest (-OR) oder einen der Reste X
- Z: einen Rest (-OR) oder einen der Reste X

Organische Photoleiter sind seit langem bekannt und werden beispielsweise in Photokopierern und Laserdruckern eingesetzt (s. z.B. EP-A 504 059). In diesen Geräten werden organische Photoleiter, die in einem neutralen Bindemittel dispergiert sind, eingesetzt. (Borsenberger, J. Phys.Chem. 1993, 11314). Derartige Photoleiterschichten haben eine typische Ladungsträgerbeweglichkeit von ca. 10⁻⁶ cm²/Vs.

Diskotisch flüssigkristalline Verbindungen wie beispielsweise Hexapentyloxytriphenylene können in der flüssigkristallinen Phase gute photoleitende Eigenschaften mit Ladungsträgerbeweglichkeiten von ca. 10⁻³ cm²/Vs aufweisen (Closs et at., Liquid Crystalls, 14 (3), 629 (1993), Bengs et al., Liquid Crystalls, 15 (5), 565 (1993) und Adam et al., Phys. Rev. Lett., 70 (4), 457 (1993). Diese Verbindungen zeigen jedoch oft erst bei höheren Temperaturen flüssigkristallines Verhalten und lassen bezüglich ihrer Kompatibilität mit Bindemitteln oder Polymerfilmen zu wünschen übrig.

Aufgabe der vorliegenden Erfindung war es, flüssigkristalline Verbindungen mit guten photoleitenden Eigenschaften bei niedrigen Temperaturen sowie guter Verarbeitbarkeit zu photoleitenden Schichten bereitzustellen.

Demgemäß wurden die eingangs definierten Derivate des Triphenylens gefunden. Außerdem wurden Verfahren zur Herstellung dieser Verbindungen, ihre Verwendung u.a. als Ladungstransportverbindungen sowie neue, für die Herstellung der Verbindungen I geeignete Zwischenprodukte gefunden.

Als Reste R kommen Alkylreste mit 2 bis 20- C-Atome in Betracht, wie Ethyl, Propyl, Butyl, Pentyl, Hexyl, Heptyl, Octyl, Nonyl, Decyl, Dodecyl, Tetradecyl oder Hexadecyl, wobei die Alkylreste mit 4 bis 16 C-Atomen bevorzugt sind. Besonders bevorzugt sind die Reste Butyl, Pentyl, Hexyl, Heptyl, Octyl und Nonyl und darunter besonders der n-Butyl- und der n-Pentylrest.

Aus Gründen der leichteren Herstellbarkeit sind die Triphenylenderivate, die gleiche Reste R tragen bevorzugt, jedoch kann für Triphenylene mit besonderen Eigenschaften auch ein Substitutionsmuster mit verschiedenen Resten R vorteilhaft sein.

Als Substituenten X kommen neben Fluor, Chlor, Brom, Jod, Nitro, Cyano und Formyl auch Azogruppen Ar-N=N- in Betracht, in denen Ar ein isoaromatischer Rest, wie Phenyl oder Naphthyl, oder ein heteroaromatischer Rest ist, der sich vom Pyrrol, Furan, Thiophen, Pyrazol, Imidazol, Oxazol, Isoxazol, Thiazol, Isothiazol, Triazol, Oxdiazol, Thiadiazol, Benzofuran, Benzthiophen, Benzimidazol, Benzoxazol, Benzthiazol, Benzisothiazol, Pyridothiophen, Pyrimidinothiophen, Thienothiophen oder Thienothiazol ableitet. Diese Reste können ihrerseits wiederum z.B. mit Alkyl-, Alkoxy, Aryl, Aryloxy, Cyano oder Nitroresten substituiert sein.

Die Diazosubstituenten Ar-N=N- bilden mit dem Triphenylenringsystem ein konjugiertes π-Elektronensystem, welches durch die Auswahl der Diazoreste variiert werden kann. Dadurch erhalten die Triphenylene interessante optische Eigenschaften z.B. bezüglich ihrer Absorption oder ihres Fluoreszenzverhaltens.

Weiterhin kommen als Substituenten X aliphatische Gruppen mit 2 bis 20 C-Atomen in Betracht, z.B. Alkylreste wie Ethyl, Propyl, Butyl, Pentyl, Hexyl, Heptyl, Octyl, Nonyl, Decyl, Dodecyl, Tetradecyl und Hexadecyl. Bevorzugt sind davon die Alkylreste mit 4 bis 16 C-Atomen, besonders bevorzugt die Reste Butyl, Pentyl, Hexyl, Heptyl, Octyl und Nonyl.

Weiterhin eignen sich als Substituenten X aliphatische Gruppen, die in ihrer Kohlenstoffkette Doppel- oder Dreifachbindungen tragen, beispielsweise Ethenyl, Propenyl, Butenyl, Pentenyl, Hexenyl, Heptenyl, Octenyl, Nonenyl, Decenyl, Dodecenyl, Tetradecenyl, Hexadecenyl, Ethinyl, Propinyl, Butinyl, Pentinyl, Hexinyl, Heptinyl, Octinyl, Noninyl, Decinyl, Dodecinyl, Tetradecinyl oder Hexadecinyl, wobei die Doppel- oder Dreifachbedingungen vorzugsweise endständig sind. Besonders bevorzugt sind aliphatische Gruppen mit Doppel- und Dreifachbindungen, welche mit dem Triphenylenkern konjugiert sind.

Besonders zu nennen sind die als Zwischenprodukte wichtigen Verbindungen, welche als Substituenten X die substituierten Alkinylreste 3-Methyl-3-Hydroxybutin-1-yl oder Trimethylsilylethin-1-yl tragen.

Als Substituenten in den aliphatischen Resten X kommen beispielsweise die Halogene, vor allem Fluor, die Hydroxygruppe, C₁-C₄-Alkoxygruppen sowie Sauerstoff in Oxofunktion in Betracht, wobei die 1-Stellung für die Oxofunktion bevorzugt ist. Reste X dieser Art sind vor allem die Alkanoylreste wie Acetyl, Trifluoracetyl, Propanoyl, Butanoyl, Pivaloyl, Pentanoyl, Hexanoyl, Heptanoyl, Octanoyl, Nonanoyl, Decanoyl, Dodecanoyl, Tetradecanoyl und Hexadecanoyl.

Neben den aliphatischen Gruppierungen kommen für X auch araliphatische Reste in Betracht. Besonders zu nennen sind aus dieser Gruppe die Benzylgruppe und die Benzoylgruppe, die ihrerseits wiederum durch verschiedene Reste substituiert sein können.

Weiterhin kommen als Substituenten X aliphatische Gruppen mit 2-20 C-Atomen und araliphatische Gruppen mit bis zu 20 C-Atomen in Betracht, die über Schwefel, über Sulfongruppen (-SO₂-) oder über Sulfonyloxygruppen (-SO₂-O-) mit dem Triphenylen verbunden sind. Dabei können die aliphatischen oder araliphatischen Reste die gleichen sein wie sie für die direkt mit ihrem C-Atom an das Triphenylen gebundenen Reste genannt sind. Bevorzugt sind auch für diese Substituenten Reste mit 4 bis 9 C-Atomen wie Butylthio, Pentylthio, Hexylthio, Heptylthio, Octylthio und Nonylthio sowie die entsprechenden Sulfonyl- oder Sulfonyloxyreste. Weiterhin kommen darunter Substituenten mit perfluorierten Alkylgruppen in Betracht, darunter besonders die Trifluormethylsulfonyloxygruppe sowie Substituenten mit hydroxysubstituierten Alkylgruppen, darunter besonders bevorzugt die Hydroxymethylsulfonylgruppe.

Die Substituenten Y und Z können einen Rest (-OR) oder einen der Reste X bedeuten, bevorzugt sind jedoch die Triphenylenderivate, in denen Y und Z einen Rest (-OR) bedeuten.

Bedeuten Y und/oder Z jedoch auch einen der Reste X, so sind die Triphenylenderivate bevorzugt, in denen X, Y und/oder Z gleiche Reste bedeuten.

Bevorzugte Triphenylenderivate I sind neben den Beispielverbindungen auch 3,6,7,10,11-Pentapentyloxytriphenylen-2-yl-pivaloat und 3,6,7,10,11-Pentapentyloxytriphenylen-2-yl-triflat.

Zur Herstellung der Triphenylenderivate geht man vorzugsweise von den hydroxysubstituierten Triphenylenen der allgemeinen Formel IIa aus 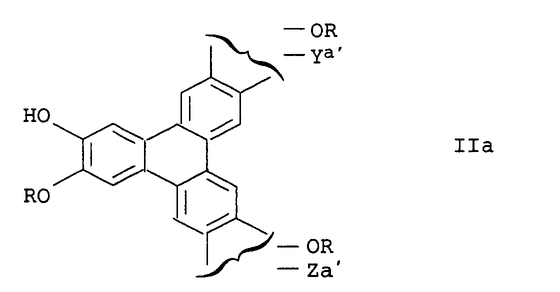 in der Y^{a'} und Z^{a'} einen Rest (-OR) oder eine Hydroxygruppe bedeuten. Die Herstellung solcher Hydroxy-Triphenylene ist beispielsweise aus Kranig et al., Adv. Materials 1, 36 (1990) oder Closs et al., J.Chem. Soc. Perkin Trans. I 829 (1995) bekannt.

Die Hydroxy-Triphenylenderivate IIa lassen sich z.B. durch Umsetzung mit einem Tetrazol und anschließende Reduktion, beispielsweise mit Wasserstoff und mit Hilfe eines Palladiumkatalysators in die entsprechenden Verbindungen der allgemeinen Formel IIh überführen 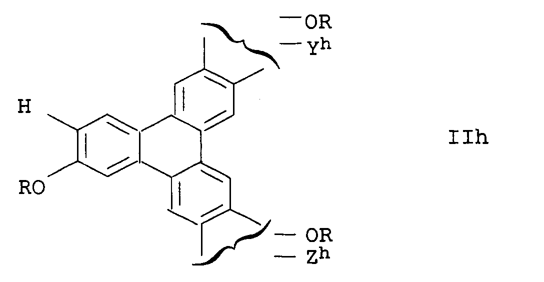 in der Y^{h} und Z^{h} einen Rest (-OR) oder Wasserstoff bedeuten. Diese Verbindungen stellen wichtige Zwischenprodukte zur Herstellung verschiedener Triphenylenderivate dar.

Durch Reaktion mit einer Verbindung IIIa

X^{a} - Nu IIIa

in der Nu einen Halogenrest wie Chlor, Brom oder Jod oder eine nukleophile Abgangsgruppe wie der Tosyl- oder Mesylrest bedeutet, läßt sich eine Vielzahl von Triphenylenen herstellen.

Triphenylenderivate, die über diesen Weg erhalten werden können, sind z.B. die Verbindungen der allgemeinen Formel Ia 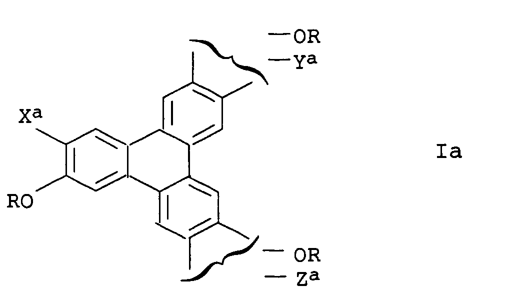 in der
- X^{a}: Fluor, Chlor, Brom, Jod, Formyl, eine Azogruppe Ar-N=N-, eine aliphatische Gruppe mit 2-20 C-Atomen oder eine araliphatische Gruppe mit bis zu 20 C-Atomen, die an einem C-Atom mit dem Triphenylen verbunden ist,
- Y^{a}: einen Rest OR oder einen der Reste X^{a} und
- Z^{a}: einen Rest OR oder einen der Reste X^{a}
bedeuten.

Zur Herstellung der Halogenverbindungen wird eine Verbindung IIh vorzugsweise direkt mit dem elementaren Halogen, z.B. Br₂ umgesetzt.

Der Formylrest sowie andere Acylgruppen können aus IIh durch Umsetzungen unter den Bedingungen der Friedel-Crafts-Acylierung erhalten werden.

Die aliphatischen und araliphatischen Reste, die am C₁-Atom unsubstituiert sind, sind beispielsweise aus den Halogeniden unter den Bedingungen der Friedel-Crafts-Alkylierung erhältlich.

Triphenylenderivate der allgemeinen Formel Ib 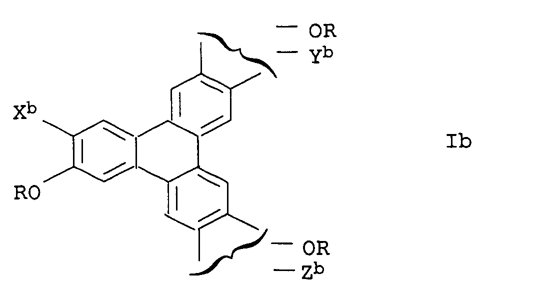 in der
- X^{b}: ein Alk-1-in-1-yl mit 2-20 C-Atomen, welches auch substituiert sein kann und
- Y^{b} und Z^{b}: ein Rest (-OR) oder ein Rest der Definition von X^{b}
bedeuten, können durch Umsetzung einer Verbindung IIh mit einem Alk-1-in X^{b}-H hergestellt werden. Die Umsetzung erfordert einen basischen Katalysator und wird daher bevorzugt in Gegenwart eines tertiären Amins durchgeführt, beispielsweise von Diisopropylamin. Vorteilhaft ist dabei weiterhin die Verwendung eines Katalysators wie Palladiumdichlorid-Kupferdiacetat-Triphenylphosphin.

Triphenylenderivate der allgemeinen Formel Ic 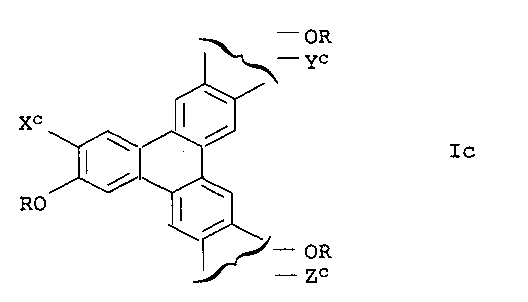 in der
- X^{c}: eine aliphatische Gruppe mit 2-20 C-Atomen oder eine araliphatische Gruppe mit bis zu 20 C-Atomen, die über eine Sulfonoxygruppierung mit dem Triphenylen verbunden ist
- Y^{c}: einen Rest (-OR) oder einen der Reste X^{c} und
- Z^{c}: einen Rest (-OR) oder einen der Reste X^{c}
bedeuten, können durch Umsetzung der Hydroxyverbindungen IIa mit einem Halogenid oder einem Anhydrid einer Sulfonsäure X^{c}-H erhalten werden. Besonders bevorzugt ist die Umsetzung mit einer äquimolaren Mischung eines Sulfonsäureanhydrids mit einem tertiären Amin wie Triethylamin. Die Reaktion wird vorteilhaft bei niedrigen Temperaturen, z.B. zwischen 0 und 10°C durchgeführt.

Zur Herstellung von Triphenylenderivaten der allgemeinen Formel Id 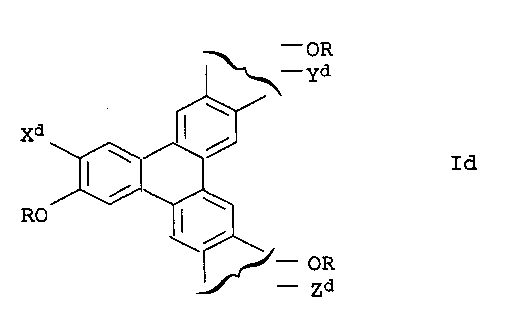 in der
- x^{d}: Cyano, eine aliphatische Gruppe mit 2-20 C-Atomen oder eine araliphatische Gruppe mit bis zu 20 C-Atomen, die über Schwefel oder eine Sulfongruppierung mit dem Triphenylen verbunden ist
- Y^{d}: ein Rest (-OR) oder einen der Reste X^{d} und
- Z^{d}: ein Rest (-OR) oder einen der Reste X^{d}
bedeuten, geht man vorteilhaft von den Bromderivaten IIbr 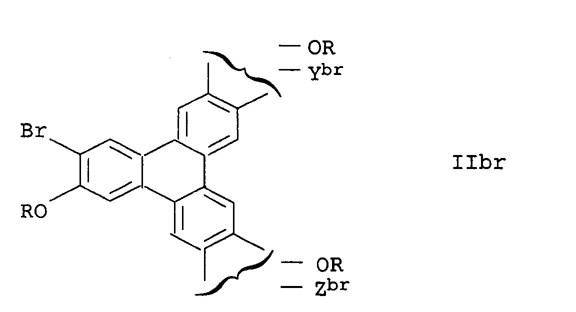 in der Y^{br} und Z^{br} einen Rest (-OR) oder Brom bedeuten, aus. Zur Herstellung der Thioether wird die Verbindung IIbr, z.B. mit einem entsprechenden Mercaptan umgesetzt. Die Umsetzung wird bevorzugt mit einer äquimolaren Mischung aus dem Mercaptan und einer sterisch gehinderten basischen Verbindung wie bevorzugt Kalium-tert.-butanolat durchgeführt.

Als Lösungsmittel wird bevorzugt ein polares aprotisches Lösungsmittel verwendet, besonders bevorzugt N-Methylpyrrolidon. Die Umsetzung gelingt besonders gut bei Verwendung eines Katalysators wie Tetra-Triphenylphosphin-Palladium (0) und wird unter Ausschuß von Sauerstoff durchgeführt.

Die Cyanoderivate des Triphenylens können leicht durch Umsetzung der Bromderivate IIbr mit Metallcyaniden, bevorzugt mit Kupfercyanid erhalten werden. Die Umsetzung wird bevorzugt in polaren aprotischen Lösungsmitteln, besonders bevorzugt in N-Methylpyrrolidon durchgeführt. Die Reaktionstemperatur ist bevorzugt die Siedetemperatur des verwendeten Lösungsmittels.

Triphenylenderivate mit Substituenten, die über eine Sulfonylgruppe mit dem Triphenylen verbunden sind, werden bevorzugt aus den entsprechenden Thioethern durch Oxidation hergestellt. Als Oxidationsmittel eignet sich besonders m-Chlorperbenzoesäure.

Alle genannten Umsetzungen führen zu Triphenylenderivaten, in denen die Substituenten Y und/oder Z, sofern sie einen Rest (-OR) bedeuten gleiche Reste X bedeuten. Die Herstellung der Triphenylenderivate, in denen X, Y und/oder Z verschiedene Reste X bedeuten, ist umständlicher, gelingt aber prinzipiell nach den gleichen Reaktionsführungen. Ausgangsverbindungen sind auch hierbei die Hydroxyverbindungen IIa. Durch unvollständige Umsetzung mit einer unterstöchiometrischen Menge eines Tetrazols und anschließender Reaktion mit Wasserstoff, wird ein Gemisch von Mono- und Dihydroxyverbindungen erhalten, welches säulenchromatographisch trennbar ist. Ausgehend von den gereinigten Mono- oder Dihydroxytriphenylenderivaten sind die gemischt substituierten Verbindungen zugänglich. Weitere Details zur Herstellung dieser Verbindungen werden von Closs et al, J. Chem. Soc. Perkin Trans. I, 829 (1995) genannt.

Die erfindungsgemäßen Verbindungen zeichnen sich im allgemeinen durch flüssigkristalline Phasen in breiten Temperaturbereichen und meist auch im Bereich der Raumtemperatur aus. Durch Mischungen verschiedener erfindungsgemäßer Triphenylenderivate oder durch Kombination mit anderen flüssigkristallinen Verbindungen lassen sich sowohl die Phasenbreite als auch der Temperaturbereich des flüssigkristallinen Zustands variieren und dadurch an die verschiedenen Verwendungszwecke anpassen.

Die erfindungsgemäßen Triphenylenderivate zeichnen sich durch gute Ladungsträgerbeweglichkeiten im flüssigkristallinen Zustand aus und eignen sich daher hervorragend als organische nichtionische Ladungstransportverbindungen. Infolge der guten Ladungstransporteigenschaft eignen sie sich zur Verwendung in verschiedenen elektrochemischen Zellen und Halbleiterbauelementen.

Durch Photoionisation können in den Triphenylenderivaten freie Ladungsträger erzeugt werden, die gut weitergeleitet werden können. Diese Eigenschaften können beispielsweise in photovoltaischen Solarzellen ausgenutzt werden, in denen die erfindungsgemäßen Verbindungen eingesetzt werden können.

Durch ihre optischen Eigenschaften, insbesondere durch ihre oft vorhandene Fluoreszenz, eignen sich die erfindungsgemäßen Verbindungen auch zur Verwendung in organischen lichtemittierenden Dioden (OLEDs). Besonders die Verbindungen, in denen Y und Z Reste (-OR) bedeuten, weisen durch die unsymetrische Substitution häufig interessante Fluoresenzbereiche und höhe Fluoresenzintensitäten auf und sind daher bevorzugt für diese Anwendungen einsetzbar.

Durch ihre gute Leitfähigkeit auch bei Raumtemperatur und die gute Kompatibilität mit neutralen Bindemitteln lassen sich die erfindungsgemäßen Triphenylene hervorragend als Photoleiter in Fotokopierern, Laserdruckern und in Laserfaxgeräten sowie in der Elektrophotographie einsetzen.

Die erfindungsgemäßen Verbindungen können in neutralen Bindemitteln dispergiert werden oder kovalent mittels vernetzungsfähiger Gruppen in den Seitenketten der Triphenylene oder auch nichtkovalent in polymere Filme integriert werden.

### Beispiele

### Beispiel 1

### 3,6,7,10,11-Pentakis(pentyloxy)triphenylen

750 mg (1,09 mmol) 2-Hydroxy-3,6,7,10,11-Pentakis(pentyloxy)triphenylen, 295 mg (1,63 mmol) 5-Chlor-1-phenyl-1H-tetrazol und 276 mg (2,0 mmol) Kaliumcarbonat wurden in 40 ml trockenem Aceton dispergiert und 18 Stunden unter Rückfluß gerührt. Nach Abdestillieren des Acetons wurde der Feststoff in Wasser und CH₂Cl₂ aufgenommen. Die organische Phase wurde zweimal mit Wasser und einmal mit gesättigter NaCl-Lösung gewaschen und dann über MgSO₄ getrocknet. Das Lösungsmittel wurde abdestilliert, und der Rückstand wurde mit 232 mg Pd-Kohle in 60 ml Ethanol/Toluol (2:1) versetzt. Die Mischung wurde 5 min mit Stickstoff gespült und dann 24 Stunden unter Rückfluß und Wasserstoffüberdruck (1 bar) gerührt. Anschließend wurde der Katalysator abfiltriert und das Lösungsmittel abdestilliert. Die Reinigung erfolgte durch Säulenchromatographie.
Schmelzpunkt: 86°C
Ausbeute: 80 %

### Beispiel 2

### 3,7,11-Tris(pentyloxy)triphenylen

Analog zu Beispiel 1 wurden 461 mg (0,93 mmol) 2,6,10-Trihydroxy-3,7,11-tris(pentyloxy)triphenylen mit 590 mg (3,27 mmol) 5-Chlor-1-phenyl-1H-tetrazol und 645 mg (4,67 mmol) Kaliumcarbonat umgesetzt. Die Hydrierung erfolgte in Tetrahydrofuran/Benzol (1:1) mit 993 mg Pd-Kohle.
Schmelzpunkt: 95°C
Ausbeute: 67 %

### Beispiel 3

### 2-Acetyl-3,6,7,10,11-Pentakis(pentyloxy)triphenylen

66 mg (0,1 mmol) 3,6,7,10,11-Pentakis (pentyloxy)triphenylen wurden bei 0°C in 2 ml Schwefelkohlenstoff gelöst und mit 20 mg (0,15 mmol) AlCl₃ und 12 mg (0,15 mmol) Acetylchlorid versetzt. Die Mischung wurde 30 min bei Raumtemperatur gerührt und dann auf gekühlte 2N HCl gegossen. Die Suspension wurde dreimal mit je 10 ml Diethylether ausgeschüttelt. Die Etherphasen wurden über MgSO₄ getrocknet, und dann wurde der Ether abdestilliert. Die Reinigung erfolgte durch Umkristallisation.
Ausbeute: 90 %

### Beispiel 4

### 2-Brom-3,6,7,10,11-Pentakis(pentyloxy)triphenylen

Zu 100 mg (0,152 mmol) 3,6,7,10,11-Pentakis(pentyloxy)triphenylen in 5 ml CH₂Cl₂ wurden innerhalb einer Stunde 27 mg (0,167 mmol) Brom in 2 ml CH₂Cl₂ bei 0°C zugetropft. Die Mischung wurde für weitere 2 Stunden gerührt, dann mit 50 ml CH₂Cl₂ versetzt und dreimal mit 5 gew.-%-iger Natriumthiosulfatlösung ausgeschüttelt. Die organische Phase wurde über MgSO₄ getrocknet, und das Lösungsmittel wurde abdestilliert. Die Reinigung erfolgte durch Säulenchromatographie.
Ausbeute: 98 %

### Beispiel 5

### 2-Cyano-3,6,7,10,11-Pentakis(pentyloxy)triphenylen

100 mg (0,136 mmol)2-Brom-3,6,7,10,11-Pentakis(pentyloxy)triphenylen und 18 mg Kupfercyamid wurden in 1,5 ml N-Methylpyrrolidon 3 Stunden unter Rückfluß gerührt. Das Gemisch wurde abgekühlt, auf Eis gegossen und dann dreimal mit CH₂Cl₂ ausgeschüttelt. Die vereinigten organischen Phasen wurden über MgSO₄ getrocknet, und anschließend wurde das Lösungsmittel abdestilliert. Die Reinigung erfolgte durch Säulenchromatographie.
Ausbeute: 95 %

### Beispiel 6

### 2-(2-Butylethinyl)-3,6,7,10,11-pentakis(pentyloxy)triphenylen

Eine Lösung aus 150 mg (0,23 mmol) 2-Brom-3,6,7,10,11-Pentakis (pentyloxy)triphenylen und 0,23 ml (2,03 mmol) 1-Hexin in 15 ml trockenem Diisopropylamin wurde 1 min mit Stickstoff gespült. Dann wurden 20 mg eines Katalysatorgemisches bestehend aus Palladiumdichlorid, Kupferdiacetat und Triphenylphosphin zugegeben, und die Mischung wurde 5 Stunden unter Rückfluß gerührt. Die Mischung wurde filtriert, und das Amin wurde abdestilliert. Die Reinigung erfolgte durch Säulenchromatographie.
Ausbeute: 93 %

### Beispiel 7

### 2,6,10-Tribrom-3,7,11-tris(pentyloxy)triphenylen

114 mg (0,236 mmol) 2,6,10-Tris(pentyloxy)triphenylen wurde analog zu Beispiel 4 mit 124 mg (0,776 mmol) Brom umgesetzt und aufgearbeitet.
Ausbeute: 92 %

### Beispiel 8

### 2,6,10-Tris(pentylthio)-3,7,11-tris(pentyloxy)triphenylen

100 mg (0,96 mmol) 1-Pentylmercaptan wurden mit 117 mg (0,96 mmol) Kalium-tert.-butanolat in 2 ml N-Methylpyrrolidon für 10 min auf 70°C erhitzt. Dann wurden 20 mg (0,028 mmol) 2,6,10-Tribrom-3,7,11-Tris(pentyloxy)triphenylen und 20 mg (0,017 mmol) Tetra-Triphenylphosphin-Palladium (0) zugegeben, und es wurde weitere 2 Stunden bei 100°C unter Stickstoff gerührt. Anschließend wurde das Gemisch auf Eis gegossen, und der Niederschlag wurde abfiltriert. Die Reinigung erfolgte durch Säulenchromatographie.
Ausbeute: 50 %

### Beispiel 9

### 3,6,7,10,11-Pentakis(pentyloxy)-2-trifluormethylsulfonyloxytriphenylen

200 mg (0,29 mmol) 2-Hydroxy-3,6,7,10,11-Pentakis(pentyloxy)triphenylen in 10 ml CH₂Cl₂ wurden tropfenweise mit einer Lösung von 0,061 ml (0,435 mmol)Triethylamin und 0,071 ml (0,435 mmol) Trifluormethylsulfonylanhydrid in 2 ml CH₂Cl₂ versetzt und 20 min bei 0°C gerührt. Nach Verdünnung mit CH₂Cl₂ wurde die Mischung dreimal mit 1N HCl und einmal mit gesättigter NaCl-Lösung ausgeschüttelt. Die organische Phase wurde über MgSO₄ getrocknet, und dann wurde das Lösungsmittel abdestilliert. Die Reinigung erfolgte durch Säulenchromatographie.
Ausbeute: 94 %

### Beispiel 10

Das Phasenverhalten der flüssigkristallinen Verbindungen der Beispiele 3 bis 9 wurde untersucht. Für den Einsatz als Ladungstransportverbindungen bzw. Photoleiter beispielsweise in Photokopierern oder Laserdruckern ist ein diskotisch-flüssigkristallines Phasenverhalten über einen breiten Temperaturbereich, besonders im Bereich von 30-50°C wünschenswert.

Die Phasenumwandlungstemperaturen wurden wie üblich mit einem Polarisationsmikroskop (Leitz Ortholux II pol) in Verbindung mit einem Mikroskopheiztisch (Mettler FP 800/82) bestimmt.

Die Abkürzungen haben folgende Bedeutung:
- cr, cr_{1,2}: = kristallin
- Dₕₒ: = hexagonal diskotisch kolumnar
- Dₓ: = höher geordnet diskotisch kolumnar
- is: = isotrop

Folgende Phasenumwandlungstemperaturen wurden für die Verbindungen nach den Beispielen 3 - 9 gemessen.

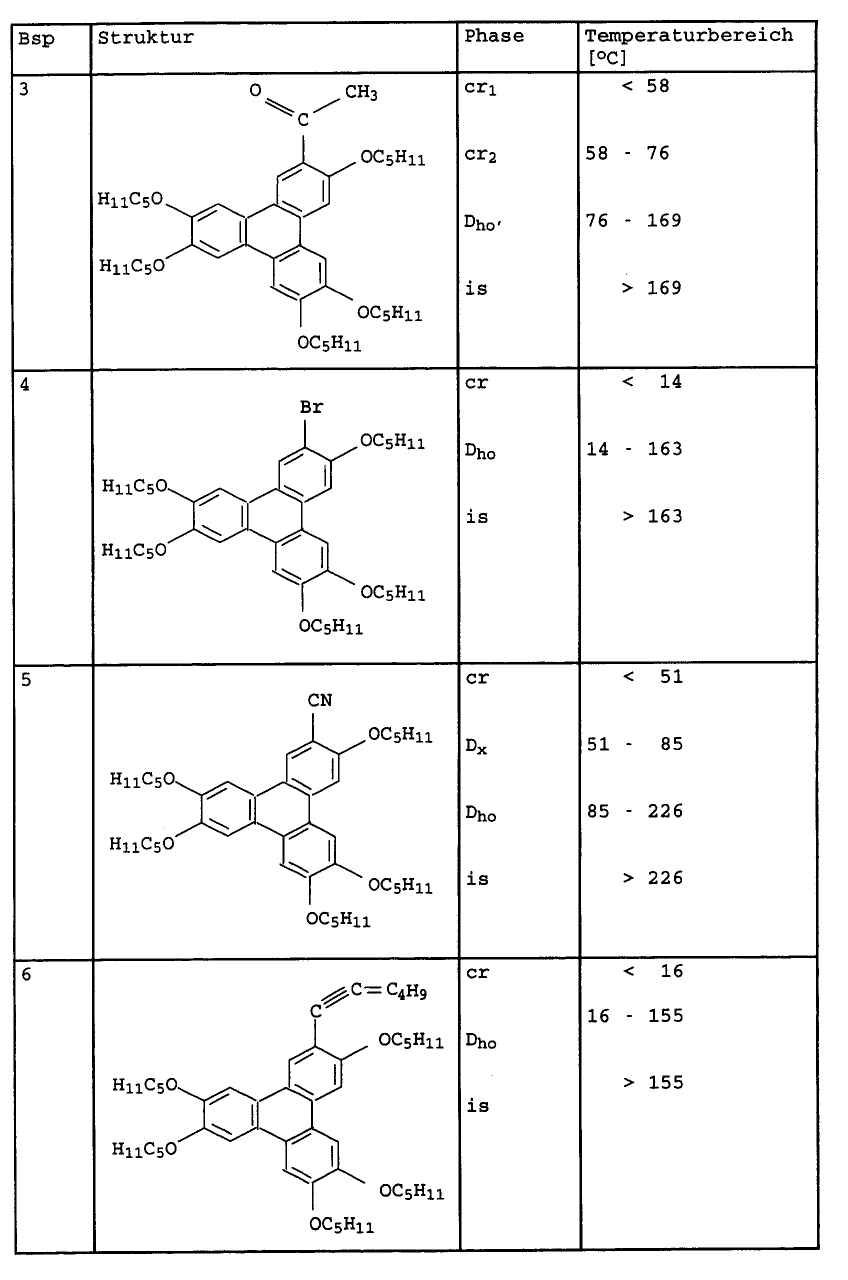

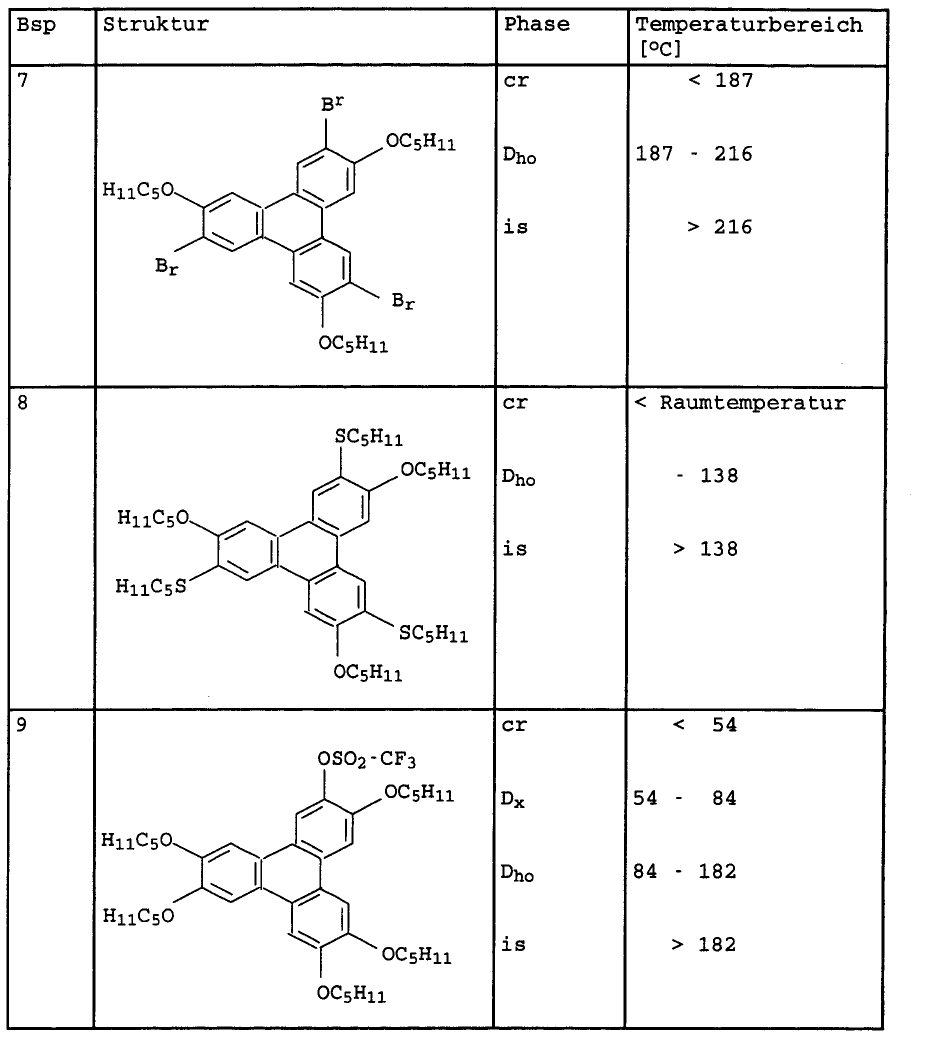

## Patentansprüche

1. Derivate des Triphenylens der allgemeinen Formel I 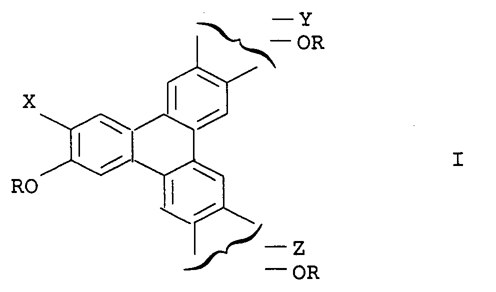 in der die Substituenten folgende Bedeutung haben:
R gleiche oder verschiedene C₂-C₂₀-Alkylreste,
X Fluor, Chlor, Brom, Jod, Nitro, Cyano, Formyl, eine Azogruppe Ar-N=N-, in der Ar für einen iso- oder heteroaromatischen Rest steht, eine aliphatische Gruppe mit 2 bis 20 C-Atomen oder eine araliphatische Gruppe mit bis zu 20 C-Atomen, die an einem C-Atom oder über Schwefel oder eine Sulfon- oder Sulfonoxygruppierung mit dem Triphenylen verbunden ist
Y einen Rest (-OR) oder einen der Reste X
Z einen Rest (-OR) oder einen der Reste X

2. Triphenylenderivate nach Anspruch 1, in denen Y und Z einen Substituenten (-OR) bedeuten.

3. Triphenylenderivate nach Anspruch 1 oder 2, in denen die Reste R gleiche oder verschiedene C₄-C₁₆-n-Alkylreste bedeuten.

4. Triphenylenderivate nach den Ansprüchen 1 bis 3, in denen die Reste R gleiche oder verschiedene C₄-C₉-n-Alkylreste bedeuten.

5. Triphenylenderivate nach den Ansprüchen 1 bis 4, in denen die Reste R gleiche Bedeutung haben.

6. Triphenylenderivate nach den Ansprüchen 1 bis 5, in denen
X Chlor, Brom, Nitro, Cyano, C₂-C₁₂-Alkinyl, C₁-C₁₂-Alkanoyl oder Ar-N=N-
bedeutet.

7. Verfahren zur Herstellung von Triphenylenderivaten der allgemeinen Formel Ia 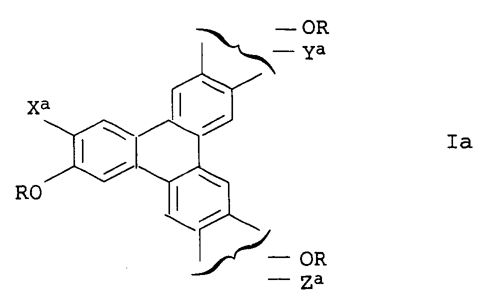 in der
X^{a} Fluor, Chlor, Brom, Jod, Formyl, eine Azogruppe Ar-N=N-, eine aliphatische Gruppe mit 2-20 C-Atomen oder eine araliphatische Gruppe mit bis zu 20 C-Atomen, die an einem C-Atom mit dem Triphenylen verbunden ist,
Y^{a} einen Rest OR oder einen der Reste X^{a} und
Z^{a} einen Rest OR oder einen der Reste X^{a}
bedeuten, dadurch gekennzeichnet, daß man ein Triphenylenderivat der allgemeinen Formel IIa 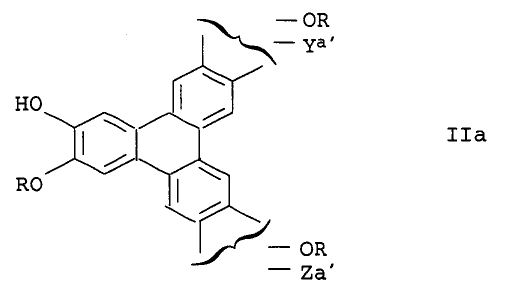 in der
Y^{a'} und Z^{a'} einen Rest OR oder eine Hydroxylgruppe bedeuten, mit Wasserstoff katalytisch reduziert und das erhaltene Zwischenprodukt IIh 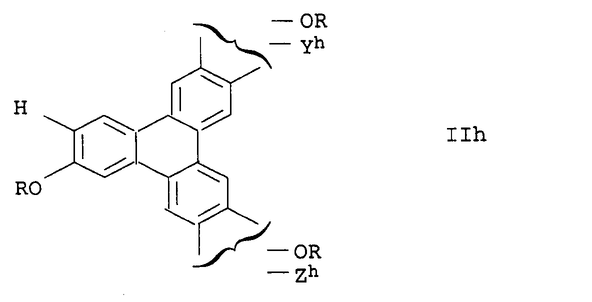 in der
Y^{h} und Z^{h} einen Rest OR oder Wasserstoff bedeuten, mit einer Verbindung IIIa
X^{a} - Nu IIIa
in der Nu einen Halogenrest oder eine nukleophile Abgangsgruppe bedeutet, umsetzt.

8. Verfahren zur Herstellung von Triphenylenderivaten der allgemeinen Formel Ib 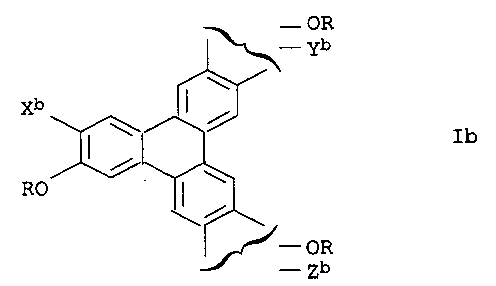 in der
X^{b} eine Alk-1-in-1-ylgruppe mit 2-20 C-Atomen, welche auch substituiert sein kann und
Y^{b} und Z^{b} Reste (-OR) oder Reste der Definition von X^{b}
bedeuten, dadurch gekennzeichnet, daß man ein Triphenylenderivat der allgemeinen Formel IIh mit einem Alkin
X^{b} -H
in Gegenwart eines tertiären Amins und eines Katalysators umsetzt.

9. Verfahren zur Herstellung von Triphenylenderivaten der allgemeinen Formel Ic 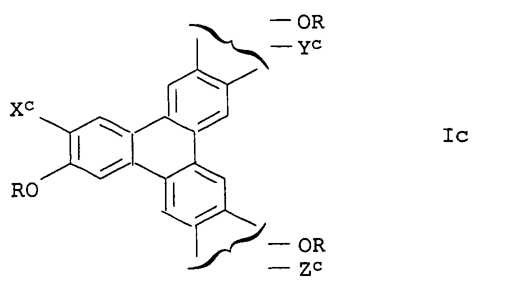 in der
X^{c} eine aliphatische Gruppe mit 2-20 C-Atomen oder eine araliphatische Gruppe mit bis zu 20 C-Atomen, die über eine Sulfonoxygruppierung mit dem Triphenylen verbunden ist
Y^{c} einen Rest (-OR) oder einen der Reste X^{c} und
Z^{c} einen Rest (-OR) oder einen der Reste X^{c}
bedeuten, dadurch gekennzeichnet, daß man ein Triphenylenderivat der allgemeinen Formel IIa mit einem Halogenid oder dem Anhydrid einer Sulfonsäure X^{c}-H umsetzt.

10. Verfahren zur Herstellung von Triphenylenderivaten der allgemeinen Formel Id 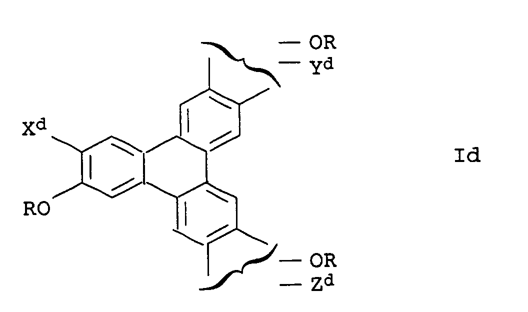 in der
x^{d} Cyano, eine aliphatische Gruppe mit 2-20 C-Atomen oder eine araliphatische Gruppe mit bis zu 20 C-Atomen, die über Schwefel oder eine Sulfongruppierung mit dem Triphenylen verbunden ist
Y^{d} ein Rest (-OR) oder einen der Reste X^{d} und
Z^{d} ein Rest (-OR) oder einen der Reste X^{d}
bedeuten, dadurch gekennzeichnet, daß man ein Triphenylenderivat IIh mit Brom zu dem Triphenylenderivat IIbr 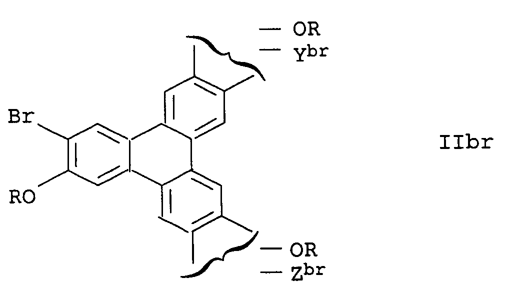 umsetzt, in der Y^{br} und Z^{br} einen Rest (-OR) oder Brom bedeuten, und anschließend, für den Fall, daß X^{d} einen aliphatischen oder araliphatischen über Schwefel mit dem Triphenylen verbundenen Rest mit bis zu 20 C-Atomen bedeutet, mit einem aliphatischen oder araliphatischen Mercaptan mit bis zu 20 C-Atomen in Gegenwart eines stark basischen Katalysators umsetzt oder,
für den Fall, daß X^{d} einen Cyanorest bedeutet, mit einem Metallcyanid umsetzt oder,
für den Fall, daß X^{d} einen aliphatischen oder araliphatischen über eine Sulfongruppierung mit dem Triphenylen verbundenen Rest mit bis zu 20 c-Atomen bedeutet, die entsprechende Thioetherverbindung mit m-Chlorperbenzoesäure zum Sulfon oxidiert.

11. Triphenylenderivate der allgemeinen Formel IIh gemäß Anspruch 7 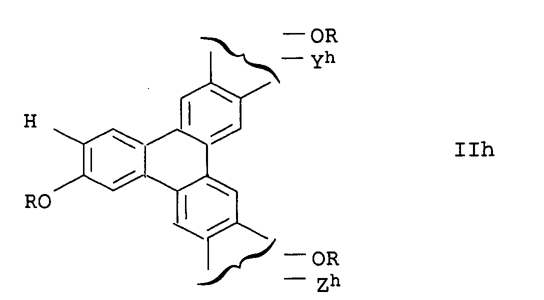 in der Y^{h} und Z^{h} Wasserstoff bedeuten.

12. Verwendung von Verbindungen gemäß den Ansprüchen 1 bis 10 als Ladungstransportmaterialien in Photokopierern, Laserdruckern und Laserfaxgeräten.

13. Verwendung der Triphenylenderivate I gemäß den Ansprüchen 1 bis 10 in elektrochemischen Zellen, photoelektrochemischen Zellen und elektronischen Bauelementen.

14. Verwendung von Verbindungen gemäß den Ansprüchen 1 bis 10 in lichtemittierenden Dioden (LEDs).
